# EUROPEAN PATENT SPECIFICATION

(11) **EP 3 946 221 B1**
(45) Date of publication and mention of the grant of the patent: **28.09.2022**
(21) Application number: 20715021.0
(22) Date of filing: 25.03.2020
(51) Int. Cl.: A61K 8/06, A61Q 19/00, A61K 8/34, A61K 8/44, A61K 8/49

(54) **MULTIPLE EMULSION COMPRISING AN OIL CONTINUOUS NANOEMULSION AND A METHOD FOR USING THE SAME**
MEHRFACHEMULSION MIT EINER KONTINUIERLICHEN ÖLNANOEMULSION UND VERFAHREN ZUR VERWENDUNG DAVON
ÉMULSION MULTIPLE COMPRENANT UNE NANOÉMULSION À PHASE HUILEUSE CONTINUE ET SON PROCÉDÉ D'UTILISATION

(30) Priority: 01.04.2019 EP 19166593
(43) Date of publication of application: 09.02.2022
(73) Proprietor: UNILEVER GLOBAL IP LIMITED, Wirral Merseyside CH62 4ZD (GB); Unilever IP Holdings B.V., 3013 AL Rotterdam (NL)
(72) Inventor: LOU, Anjing, Trumbull, CT Connecticut 06611 (US)
(74) Representative: James, Helen Sarah
(86) International application number: PCT/EP2020/058433
(87) International publication number: WO 2020/200979

(56) References cited:
- WO-A1-01/28502
- WO-A1-03/039724
- CN-A- 109 199 878
- CN-A- 109 276 515
- US-A1- 2010 161 029

## Description

### Field of the invention

The present invention is directed to a multiple emulsion comprising an oil continuous nanoemulsion and a method for using the same. More particularly, the invention is directed to a multiple emulsion that is water continuous but contains, as an internal phase, an oil continuous nanoemulsion.

### Background of the invention

Use of nanoemulsions is becoming increasingly popular in beauty and personal care compositions. Nanoemulsions are generally stable and have a high surface area in view of their unit volume. Nanoemulsions can carry actives in their water and oil phases and are desirable since their size helps enhance penetration of active through the skin. Notwithstanding benefits associated with emulsions, especially nanoemulsions, problems in general with emulsions often concern the inability to include multiple actives as well as high levels of humectants, like glycerine, which often impede the solubility of important actives in water. Moreover, in compositions where water content is high, significant amounts of preservatives are required for microbiological stability.

It has been unexpectedly discovered that when oil continuous nanoemulsions are present as the internal phase in a water continuous multiple emulsion, hydrophilic actives can be separated and end use compositions with high water content can be made to deliver good sensory and moisturization benefits. It has also been discovered that when multiple emulsions having an oil continuous nanoemulsion as the internal phase are prepared, the resulting multiple emulsion requires less preservative for microbiological stability.

There is an increasing interest to deliver compositions with actives and moisturizing benefits to consumers and without comprising high levels of preservative.

This invention, therefore, is directed to a multiple emulsion comprising an oil continuous nanoemulsion and a method for using the same. The multiple emulsion of the present invention, surprisingly, can have a high concentration of hydrophilic benefit agent in its water phases, delivers superior moisturizing benefits, and does not require high levels of preservative for microbiological stability. Such multiple emulsions are stable, have good sensory properties (consistent with traditional oil-in-water emulsions free of glycerine) and are non-sticky even when glycerine is present. The invention also is directed to a method using such multiple emulsion.

### Additional Information

Efforts have been disclosed for making emulsions. In U.S. Published Patent Application No. 2017/0112764A1, nanoemulsions having reversible continuous and dispersed phases are described.

Even other efforts have been disclosed for making emulsions. In World Application WO 03/039724 A1, multiple emulsions comprising particles of nanometric dimensions are described.

Still other efforts have been disclosed for making emulsions. In US Patent No. 5,589,177 rinse-off water-in-oil multiple emulsion compositions are described.

CN109276515 A discloses W/O/W multiple emulsions comprising humectants. The internal aqueous phase comprises a cosolvent such as 1,3-butanediol, lactitol, xylitol or glycerin. The examples show compositions comprising a low HLB surfactant for the W/O primary emulsion, high HLB surfactant for the external aqueous phase and particle sizes of the primary emulsion of 850nm or below.

CN109199878 A discloses a whitening sunscreen W/O/W multiple emulsion comprising water soluble humectants in both aqueous phases, wherein the W/O primary emulsion is a nanoemulsion.

US2010161029 A1 discloses W/O/W multiple emulsions wherein the water-in-oil (nano)emulsion comprises an emulsifier with an HLB of less than 8 and the external phase comprises an emulsifier with an HLB of more than 8, and wherein the external phase makes up 25 to 70% of the emulsion. The compositions may also comprise moisturisers such as glycerol.

WO0128502 A1 discloses stable W/O/W emulsions wherein the water in oil phase comprises a surfactant having an HLB of 10 or less, and comprising skin benefit agents including humectants such as glycerin and water-soluble vitamin A, E and D. There is no mention of particle sizes of the W/O primary emulsion.

None of the additional information above describes a multiple emulsion and method for using a multiple emulsion as set forth in the present claims.

### Summary of the invention

The present invention is directed to a cosmetic composition comprising a multiple emulsion comprising:
(a) an internal phase comprising a water-in-oil nanoemulsion comprising emulsifier, the nanoemulsion having a particle size from 100 to 850 nm, and further comprising from 0.0 to 15% by weight oil soluble benefit agent, from 0.0 to 15% by weight water soluble benefit agent and from 0.0 to 20% by weight humectant based on total weight of the nanoemulsion; and
(b) an external aqueous phase comprising water, from 0.0 to 20% by weight humectant and from 0.0 to 15% by weight water soluble benefit agent based on total weight of the external aqueous phase,
the cosmetic composition comprising from 20 to 65% (preferably, 40 to 60%) by weight nanoemulsion based on total weight of the cosmetic composition, with the proviso that the nanoemulsion and external aqueous phase do not simultaneously have 0.0% by weight humectant,
and in which the water phase of the nanoemulsion of the multiple emulsion, includes water insoluble benefit agents selected from insoluble amino acids selected from phenylalanine, tyrosine, tryptophan, cystine or mixtures thereof; and in which the water insoluble benefit agents make up from 0.02 to 5% by weight of the total weight of the multiple emulsion.

In a further aspect, the invention is directed to the use of the multiple emulsion and cosmetic composition of the first and second aspects of the invention to improve a cosmetic skin characteristic.

All other aspects of the present invention will more readily become apparent from the description and examples which follow.

Skin, as used herein, is meant to include skin on the arms (including underarms), face, feet, neck, chest, hands, legs, buttocks and scalp (including hair). Particle size, as it relates to the multiple emulsion and nanoemulsion means the volume average diameter of the water droplets in microns or nanometers, respectiviely. Water droplet size may be measured with a commercially available Malvern Mastersizer. Cosmetic composition is a composition for topical application and includes a cream, lotion, balm, serum, gel, mousse, aerosol, deodorant, antiperspirant, shampoo, conditioner, make-up or personal wash, including bars and liquids. Such a composition can be the multiple emulsion of this invention or the multiple emulsion having additional ingredients added thereto such as oils (like silicone, fish or mineral oils), fragrances, benefit agents (like Vitamin B₃, resorcinols and retinoids) and/or colorants. Benefit agent (or active), as herein defined, is a water soluble or insoluble component that delivers a benefit to skin after being topically applied. Water insoluble means having a solubility in water of no more than 0.05% by weight at 25°C, atmospheric pressure and neutral pH. For insoluble benefit agents like certain amino acids (e.g., cystine), the pH of water may be adjusted to induce solubility of such a solute in water. Neutral pH as used herein means having a pH from 6.0 to 7.5. Viscosity, as used herein, is taken with a Brookfield helipath TD, at 4 rpm for 1 minute unless noted otherwise. Multiple emulsion, as used herein, means the water continuous (water-in-oil in water) emulsion comprising the nanoemulsion of this invention. Such a multiple emulsion typically has within its internal phase an oil droplet size from 1 to 35 microns, including all ranges subsumed therein. In another embodiment, the cosmetic composition of this invention is a leave-on skin lotion, cream or liquid personal wash composition. In still another embodiment, the cosmetic composition is a lotion or cream for topically applying and leaving on skin. In the absence of explicitly stating otherwise, all ranges described herein are meant to include all ranges subsumed therein. The term comprising is meant to encompass the terms consisting essentially of and consisting of. For the avoidance of doubt, and by illustration, an oil continuous nanoemulsion of this invention comprising oil, water, emulsifier and benefit agent is meant to include a nanoemulsion consisting essentially of the same and a nanoemulsion consisting of the same. Except in the operating comparative examples, or where otherwise explicitly indicated, all numbers in this description indicating amounts or ratios of materials or conditions and/or physical properties of materials and/or use are to be understood as modified by the word "about".

### Detailed description of the invention

As to the oil continuous nanoemulsions used in the multiple emulsion of the present invention, the same typically comprise from 30 to 70%, and in another embodiment, from 35 to 65%, and in still another embodiment, from 40 to 60% by weight water, based on total weight of the nanoemulsion and including all ranges subsumed therein. As to the oil in the nanoemulsion, oil typically makes up from 25 to 70%, and in another embodiment, 30 to 65%, and still in another embodiment, 35 to 60% by weight of the nanoemulsion, based on total weight of the nanoemulsion and including all ranges subsumed therein. Such nanoemulsions typically have a particle (water droplet) size from 100 to 850, and in another embodiment, from 150 to 800, and still in another embodiment, from 200 to 750 nanometers, including all ranges subsumed therein.

The oil for use in this invention within the nanoemulsion is limited only to the extent that the same is a liquid at room temperature (or a liquid after heating to about 35°C) and suitable for use in a topical composition applied to skin.

Illustrative examples of the oils suitable for use include silicone oils.

Silicone oils may be divided into the volatile and non-volatile variety. The term "volatile" as used herein refers to those materials which have a measurable vapor pressure at ambient temperature. Volatile silicone oils are preferably chosen from cyclic or linear polydimethylsiloxanes containing from 3 to 9, and preferably, from 4 to 5 silicon atoms.

Nonvolatile silicone oils useful in this invention include polyalkyl siloxanes, polyalkylaryl siloxanes and polyether siloxane copolymers. Such essentially non-volatile polyalkyl siloxanes useful herein include, for example, polydimethylsiloxanes (like dimethicone) with viscosities of from 5 to 100,000 centistokes at 25°C.

An often preferred silicone source is a cyclopentasiloxane and dimethicone solution.

Suitable esters for use to make emulsion in this invention include:
(1) Alkenyl or alkyl esters of fatty acids having 10 to 20 carbon atoms like isopropyl palmitate, isopropyl isostearate, isononyl isonanonoate, oleyl myristate, isopropyl myristate, oleyl stearate, and oleyl oleate;
(2) Ether-esters such as fatty acid esters of ethoxylated fatty alcohols;
(3) Polyhydric alcohol esters such as ethylene glycol mono- and di-fatty acid esters, diethylene glycol mono- and di-fatty acid esters, polyethylene glycol (200-6000) mono- and di-fatty acid esters, propylene glycol mono- and di-fatty acid esters, polypropylene glycol 2000 monooleate, polypropylene glycol 2000 mono stearate, ethoxylated propylene glycol monostearate, glyceryl mono- and di-fatty acid esters, polyglycerol poly-fatty esters, ethoxylated glyceryl mono-stearate, 1,3-butylene glycol monostearate, 1,3-butylene glycol distearate, polyoxyethylene polyol fatty acid ester, sorbitan fatty acid esters, and polyoxy-ethylene sorbitan fatty acid esters;
(4) Sterol esters, of which soya sterol and cholesterol fatty acid esters are examples thereof.

Still other oils that may be used in this invention include triglycerides (animal and/or vegetable) like soybean oil, sunflower oil, coconut oil, palm kernel oil, castor oil, rapeseed oil, palm oil, grape seed oil, caprylic/capric triglyceride, safflower oil, fish oil or mixtures thereof.

Even other oils suitable for use include mineral oil, jojoba oil, isoparaffins, C₁₂-C₁₅ alkyl benzoates, polyalphaolefins, isohexadecane, petrolatum, mixtures thereof (including with those oils above) or the like. In an embodiment of the invention, soybean and sunflower oil are the triglyceride oils used.

In still another embodiment, caprylic capric triglyceride is another oil suitable for use in the namoemulsions of the present invention.

Adjusters suitable to modify the pH of the aqueous phases in the multiple emulsion of this invention may be used. Such pH adjusters include triethylamine, NaOH, KOH, H₂SO₄, HCl, C₆ H₈ O₇ (i.e., citric acid) or mixtures thereof. The pH adjusters are added at amounts such that the resulting pH of the multiple emulsion of this invention is from 5 to 7.5. In one embodiment, the pH is from 6 to 7.5 and in still another embodiment from 6 to 7.

In an embodiment of this invention, it is particularly noted that when benefit agent is an amino acid, like cystine, an oil continuous macroemulsion having a pH from 10 to 14 with amino acid dissolved therein is combined with a macroemulsion having a pH from 2 to 5. The macroemulsions are mixed and sheared to produce nanoemulsions suitable for use as the internal phase of the water continuous multiple emulsion of this invention.

The water phase pH values of the desired nano- and multiple emulsion of the present invention are assessed by using conventional instrumentation such as a pH meter made commercially available from Thermo Scientific^{®}.

The emulsifiers suitable for use in this invention to make nanoemulsion typically have an HLB from 2.5 to less than 8.0, and preferably, from 3 to 7.0, and most preferably, from 3 to 6.5, including all ranges subsumed therein.

Illustrative examples of the types of emulsifiers that are suitable for use to make the oil continuous nanoemulsion of this invention are propylene glycol isostearate, glycol stearate sorbitan sesquioleate, lecithin, oleth-2, stearth-2, ceteth-2 glyceryl stearate, PEG-30 dipolyhydroxystearate.

Still other emulsifiers suitable for use include glycol distearate, glyceryl oleate, sorbitan monooleate, sorbitan tristearate, sorbitan trioleate, sorbitan monopalmitate, lauryl PEG-10, (trimethylsiloxy)silylethyl dimethicone (Dow Corning^{®} ES-5300) or mixtures thereof.

Illustrative examples of the types of emulsifiers that are suitable for use to make the water continuous emulsion comprising the nanoemulsion of the present invention are polysorbate 85, laureth-4, sodium laurate, cetearyl glucoside, PEG-8 oleate, Tween 20, Tween 40, oleth-10 or mixtures thereof.

In the oil continuous nanoemulsions and the water continuous multiple emulsions of this invention, emulsifiers typically and independently make up from 1.0 to 10, and preferably, from 1.2 to 8, and most preferably, from 1.5 to 7.5% by weight of each emulsion (i.e., both the nano- and water continuous), including all ranges subsumed therein.

The external phase of the water continuous emulsion typically makes up from 25 to 70% by weight of the water-in-oil in water emulsion. In one embodiment, the external phase makes up from 35 to 65% by weight of the water-in-oil in water emulsion. In still another embodiment, the external phase makes up from 40 to 60% by weight of the water-in-oil in water emulsion.

As to the optional skin benefit agents suitable for use in this invention, the same are limited only to the extent that they are capable of being topically applied, and suitable to dissolve in either the oil or water phase of the multiple emulsion of this invention. It is understood that solubility of a benefit agent in the water phase may be impacted by modifying or regulating the pH of the water phase with the adjusters herein described.

Illustrative examples of the benefit agents suitable to include in the water phases of the multiple emulsions are acids, like amino acids, such as arginine, valine or histidine. Additional water soluble benefit agents suitable for use include vitamin B₂, niacinamide (vitamin B₃), vitamin B₆, vitamin C, mixtures thereof or the like. Water soluble derivatives of such vitamins may also be employed. For instance, vitamin C derivatives such as ascorbyl tetraisopalmitate, magnesium ascorbyl phosphate and ascorbyl glycoside may be used alone or in combination with each other. Other water soluble benefit agents suitable for use in the water phases of the multiple emulsions of this invention include 4-ethyl resorcinol, extracts like sage, aloe vera, green tea, grapeseed, thyme, chamomile, yarrow, cucumber, liquorice or rosemary extract or mixtures thereof. Water soluble sunscreens like ensulizole may also be used. Total amount of water soluble benefit agents (including mixtures) when present in the water phases of the emulsions (i.e., nano- and multiple) of the present invention may range from 0.0 to 15%, preferably from 0.001 to 10%, optimally from 0.01 to 6% by weight, based on total weight of the nanoemulsion and water phase of the multiple emulsion (as the case may be) and including all ranges subsumed therein.

It is also within the scope of the present invention to include, in the water phase of the nanoemulsion of the multiple emulsion, water insoluble benefit agents such as insoluble amino acids like phenylalanine, tyrosine, tryptophan, cystine or mixtures thereof by enhancing their solubility with water pH modifications. In one embodiment, insoluble benefit agent makes up from 0.02 to 5%, and in another embodiment, from 0.1 to 3%, and in still another embodiment, from 0.3 to 2% by weight of the total weight of the multiple emulsion, including all ranges subsumed therein.

When preparing nanoemulsion having insoluble benefit agent, (e.g., cystine), typically a first water-in-oil macroemulsion is made having a pH from 10-14 in order to solubilize insoluble benefit agent. The same is combined with a second water-in-oil macroemulsion having a pH from 2.5 to 4 and no benefit agent. The macroemulsions are mixed (high pH to low pH in a weight ratio of 75:30 to 50:50 or 70:30 to 60 to 40, including all ratios subsumed therein). The resulting mixture of macroemulsion is homogenized or sheared under conditions as herein described to produce oil continuous nanoemulsion suitable for use as the internal phase of the multiple emulsion of the present invention.

It is also within the scope of the present invention to optionally include an oil soluble benefit agent in the oil phase of the nanoemulsions. The only limitation with respect to such oil soluble benefit agent is that the same is suitable to provide a benefit to skin when topically applied.

Illustrative examples of the types of oil soluble benefit agents that may optionally be used in this invention include vitamins like Vitamin A, D, E and K (and their oil soluble derivatives), sunscreens like ethylhexylmethoxycinnamate, bis-ethyl hexyloxyphenol methoxyphenol triazine, 2-ethylhexyl-2-cyano-3,3-diphenyl-2-propanoic acid, drometrizole trisiloxane, 3,3,5-trimethyl cyclohexyl 2-hydroxybenzoate, 2-ethylhexyl-2-hydroxybenzoate or mixtures thereof.

Other optional oil soluble benefit agents suitable for use include resorcinols like 4-hexyl resorcinol, 4-phenylethyl resorcinol, 4-cyclopentyl resorcinol, 4-cyclohexyl resorcinol 4-isopropyl resorcinol or a mixture thereof. Also, 5-substituted resorcinols like 4-cyclohexyl-5-methylbenzene-1,3-diol, 4-isopropyl-5-methylbenzene-1,3-diol, mixtures thereof or the like may be used. The 5-substituted resorcinols, and their synthesis are described in commonly assigned U.S. Published Patent Application No. 2016/0000669A1.

Even other oil soluble actives suitable for use include omega-3 fatty acids, omega-6 fatty acids, climbazole, farnesol, ursolic acid, myristic acid, geranyl geraniol, oleyl betaine, cocoyl hydroxyethyl imidazoline, hexanoyl sphingosine, 12-hydroxystearic acid, petroselinic acid, conjugated linoleic acid, terpineol, thymol mixtures thereof or the like.

In an embodiment of the invention, the optional oil soluble benefit agent used is a retinoic acid precursor.

In one embodiment of the invention, the retinoic acid precursor is retinol, retinal, retinyl propionate, retinyl palmitate, retinyl acetate or a mixture thereof. Retinyl propionate, retinyl palmitate and mixtures thereof are typically preferred.

Still in another embodiment, the retinoic acid precursor suitable for use is hydroxyanasatil retinoate made commercially available under the name Retextra^{®} as supplied by Molecular Design International. The same may be used in a mixture with the oil soluble actives described herein.

When the optional (i.e., 0.0 to 15% by weight) oil soluble active is used in the oil phase of the nanoemulsion of this invention, it typically makes up from 0.001 to 15%, and in another embodiment, from 0.05 to 7.0, and in yet another embodiment, from 0.1 to 5% by weight of the total weight of the nanoemulsion in the multiple emulsion of the invention and including all ranges subsumed therein.

Preservatives can desirably be incorporated into the water phases of the nanoemulsion and exterior phase of the multiple emulsion of this invention to protect against the growth of potentially harmful microorganisms, although it is within the scope of the invention for the such emulsions to be preservative free. Suitable traditional preservatives for use in this invention are alkyl esters of para-hydroxybenzoic acid. Other preservatives include hydantoin derivatives, propionate salts, and a variety of quaternary ammonium compounds. Cosmetic chemists are familiar with appropriate preservatives and routinely choose them to satisfy the preservative challenge test and to provide product stability. Particularly preferred preservatives are iodopropynyl butyl carbamate, phenoxyethanol, 1,2-octanediol, hydroxyacetophenone, ethylhexylglycerine, hexylene glycol, methyl paraben, propyl paraben, imidazolidinyl urea, sodium dehydroacetate and benzyl alcohol. The preservatives should be selected having regard for the use of the composition and possible incompatibilities between the preservatives and other ingredients in the emulsion. Preservatives are preferably employed in amounts ranging from 0.01% to 2% by weight of the total weight of the multiple emulsion, including all ranges subsumed therein. Combinations of 1,2-octanediol and phenoxyethanol, or iodopropynyl butyl carbamate and phenoxyethanol are preferred, with phenoxyethanol and 1,2-octanediol, collectively and preferably, making up less than 1.8% by weight of the total weight of the emulsion or end use composition of the present invention. Also preferred is a preservative system with hydroxyacetophenone alone or in a mixture with other preservatives.

Thickening agents are suitable for use in the emulsions of the present invention. Particularly useful are the polysaccharides. Examples include fibers, starches, natural/synthetic gums and cellulosics. Representative of the starches are chemically modified starches such as sodium hydroxypropyl starch phosphate and aluminum starch octenylsuccinate. Tapioca starch is often preferred, as is maltodextrin. Suitable gums include xanthan, sclerotium, pectin, karaya, arabic, agar, guar (including Acacia senegal guar), carrageenan, alginate and combinations thereof. Suitable cellulosics include hydroxypropyl cellulose, hydroxypropyl methylcellulose, ethylcellulose, sodium carboxy methylcellulose (cellulose gum/carboxymethyl cellulose) and cellulose (e.g. cellulose microfibrils, cellulose nanocrystals or microcrystalline cellulose). Sources of cellulose microfibrils include secondary cell wall materials (e.g. wood pulp, cotton), bacterial cellulose, and primary cell wall materials. Preferably the source of primary cell wall material is selected from parenchymal tissue from fruits, roots, bulbs, tubers, seeds, leaves and combination thereof; more preferably is selected from citrus fruit, tomato fruit, peach fruit, pumpkin fruit, kiwi fruit, apple fruit, mango fruit, sugar beet, beet root, turnip, parsnip, maize, oat, wheat, peas and combinations thereof; and even more preferably is selected from citrus fruit, tomato fruit and combinations thereof. A most preferred source of primary cell wall material is parenchymal tissue from citrus fruit. Citrus fibers, such as those made available by Herbacel^{®} as AQ Plus can also be used as source for cellulose microfibrils. The cellulose sources can be surface modified by any of the known methods including those described in Colloidal Polymer Science, Kalia et al., "Nanofibrillated cellulose: surface modification and potential applications" (2014), Vol 292, Pages 5-31.

Synthetic polymers are yet another class of effective thickening agent. This category includes crosslinked polyacrylates such as the Carbomers, polyacrylamides such as Sepigel^{®} 305 and taurate copolymers such as Simulgel^{®} EG and Aristoflex^{®} AVC, the copolymers being identified by respective INCI nomenclature as Sodium Acrylate/Sodium Acryloyldimethyl Taurate and Acryloyl Dimethyltaurate/Vinyl Pyrrolidone Copolymer. Another preferred synthetic polymer suitable for thickening is an acrylate-based polymer made commercially available by Seppic and sold under the name Simulgel INS100. Calcium carbonate, fumed silica, and magnesium-aluminum-silicate may also be used.

Amounts of the thickening agent, when used, may range from 0.001 to 20%, and preferably, from 0.1 to 15%, and most preferably, from 0.2 to 10% by weight of the multiple emulsion, based on total weight of the multiple emulsion and including all ranges subsumed therein. Maltodextrin, xanthan gum, and carboxymethyl cellulose are the often preferred.

Fragrances, fixatives, chelators (like EDTA), salts (like NaCI) and exfoliants may optionally be included in multiple emulsions of the present invention. Each of these substances may range from about 0.03 to about 5%, preferably between 0.1 and 3% by weight of the total weight of multiple emulsion, including all ranges subsumed therein.

Conventional humectants may be employed as additives in the multiple emulsion of the present invention (to the nanoemulsion and/or external water phase of the multiple emulsion) to assist in moisturizing skin when such emulsions are topically applied. These are generally polyhydric alcohol type materials. Typical polyhydric alcohols include glycerol (i.e., glycerine or glycerin), propylene glycol, dipropylene glycol, polypropylene glycol, polyethylene glycol, sorbitol, hydroxypropyl sorbitol, hexylene glycol, 1,3-butylene glycol, isoprene glycol, 1,2,6-hexanetriol, ethoxylated glycerol, propoxylated glycerol and mixtures thereof. Most preferred is glycerin, propylene glycol or a mixture thereof. The amount of humectant employed may range anywhere from 0.0 to 35% by weight of the total weight of the external water phase of the multiple emulsion and/or the nanoemulsion. Often, humectant makes up from 0.0 to 20%, and preferably, from 0.001 to 15% by weight (most preferably, from 2 to 12% by weight) of the total weight of the external water phase of the multiple emulsion and/or the total weight of the nanoemulsion. When humectant is present at 15% by weight or more in both the external water phase and the nanoemulsion, in one embodiment the total weight of preservative in the multiple emulsion does not exceed 1.3% (more preferably from 0.15 to 0.65%) by weight, based on total weight of the multiple emulsion.

When making the nanoemulsion of the present invention, the desired ingredients may be mixed to produce water and oil phases. The same may be mixed under moderate shear with emulsifier under atmospheric conditions with temperature being from ambient to 85°C. Mixing to make a resulting pre-nanoemulsion (i.e., macroemulsion, 1-20 microns) can be done with a magnetic stir bar or may be accomplished in a commercially available mixer equipped with, for example, an impeller (e.g., turbine or anchor), or a rotor/stator high shear mixer made commercially available by suppliers like Esco-Labs, AG, Silverson^{®} or Charles Ross & Son. The shear rate for mixing can vary and is preferably set such that the resulting pre-nanoemulsion is not aerated to the point of displaying visual air pockets. Often, mixing to make the pre-nanoemulsion is set with a stirrer or scraper at 35 to 500, and preferably, at 40 to 250, and most preferably, at 45 to 150 rpm. Shearing with a rotor/stator to make the desired oil continuous nanoemulsion is typically accomplished with rotation being set from 1000 to 8000, and preferable, from 2000 to 7000, and most preferably, from 2500 to 6,250 rpm, including all ranges subsumed therein.

The resulting pre-nanoemulsion typically has a viscosity from 1,000 to 65,000 cps, and preferably, from 2,000 to 45,000 cps, and most preferably, from 5,000 to 35,000 cps, including all ranges subsumed therein, where the viscosity of the macroemulsions is measured with a Brookfield (DV-1+) Viscometer, temperature 25°C and set at 20 RPM, RV6 for 30 seconds.

Additionally, the oil continuous nanoemulsion may be made by feeding the resulting pre-nanoemulsion (or mixtures of pre-nanoemulsions as is the case when the benefit agent is an amino acid) may be fed after premixing or simultaneously to a high shear mixer (like a rotor/stator) mixer or a homogenizer in order to produce the desired water-in-oil nanoemulsions of the present invention. Such nanoemulsions again have a particle droplet size from 100 to 850 nm, including all ranges subsumed therein. Oil continuous nanoemulsion production is surprisingly achieved after one pass through a homogenizer, (when a homogenizer is selected) with pressure set from 50 to 2000, and preferably, from 250 to 1000, and most preferably, from 300 to 800 psi, including all ranges subsumed therein. Preferred devices for making the nanoemulsions of the present invention are the Silverson^{®} rotor/stator mixer or homogenizer (such as a Sonic Sonolator). In an especially preferred embodiment, and when a homogenizer is used, pressure is set from 50 to 1000 psi. The viscosity of the resulting nanoemulsions will fall within the range of 750 to 55,000 cps, preferably, from 1,200 to 40,000 cps, and most preferably, from 1,500 to 30,000 cps, including all ranges subsumed therein.

Water with or without humectant is combined with the resulting oil continuous nanoemulsion (and emulsifier having an HLB of 8 or higher) and mixed under moderate shear to produce the multiple emulsion of the present invention. The multiple emulsion will have a viscosity within the ranges of a conventional oil-in-water emulsion prepared with oil and not a water-in-oil emulsion. Often, the oil droplet size of the internal phase of multiple emulsion is from 1 to 35, and preferably, from 2 to 30, and most preferably from 8 to 20 microns, including all ranges subsumed therein. The viscosity of the multiple emulsion is typically from 1,000 to 60,000 cps, and in another embodiment from 3,000 to 55,000 cps, including all ranges subsumed therein. In another embodiment, the weight ratio of water to humectant in the external aqueous phase of the present invention is from 70:30 to 55:45, including all ratios subsumed therein.

The multiple emulsions of the present invention may be used by a consumer for topical application to the body, especially the hair or skin, most preferably for use to moisturize skin.

The packaging for the multiple emulsions of this invention is typically a bottle, tube or jar. Other suitable packages include blister pack or sachets. The multiple of the present invention may also be dispensed from automatic dispensers or packaging pressurized with propellant.

The Examples provided are to facilitate an understanding of the invention. They are not intended to limit the scope of the claims.

**Example 1** Nanoemulsion, 1% cystine (amino acid as benefit agent), oil continuous and pH 7.

High pH (pH 12) pre-nanoemulsion and low pH (pH 2.5) pre-nanoemulsion were prepared separately. When preparing the high pH pre-nanoemulsion (Table 1a), all ingredients in the aqueous phase were combined in a mixing vessel and mixed (at room temperature and with moderate shear) with a magnetic stir bar, resulting in a transparent mixture. The oil phase was prepared by combining and mixing ingredients (also at moderate shear and room temperature) in a separate mixing vessel equipped with an overhead stirrer. Mixing was terminated when the resulting mixture was clear. The aqueous solution was subsequently and gradually added to the mixing vessel with the oil phase while agitation/stirring was provided to mix the two phases. Stirring was stopped after a uniform mixture was obtained and the oil continuous macroemulsion had particle size of about 10 microns.

**Table 1a. High pH pre-nanoemulsion**

| | wt%* |
|---|---|
| **Aqueous phase** | |
| DI water | Balance |
| NaOH | 0.7 |
| NaCl | 0.4 |
| EDTA | 0.4 |
| Cystine | 1.4 |
| | |

| **Oil phase** | |
|---|---|
| CCT** | 36.6 |
| DC ES-5300*** | 5.8 |

| | |
|---|---|
| * based on total weight of high pH macroemulsion ** Caprylic Capric Triglyceride *** Silicone Emulsifier, Dow Corning | |

Low pH pre-nanoemulsion, about 10 microns, (Table 1b) was prepared in a manner similar to the one described for the high pH pre-nanoemulsion described in this example.

**Table 1b. Low pH Macroemulsion**

| | wt%* |
|---|---|
| **Aqueous phase** | |
| DI water | Balance |
| Citric acid | 2.9 |
| | |

| **Oil phase** | |
|---|---|
| CCT** | 37.1 |
| DC ES-5300*** | 5.8 |

| | |
|---|---|
| * based on total weight of low pH macroemulsion ** Caprylic Capric Triglyceride *** Silicone Emulsifier, Dow Corning | |

The resulting high pH and low pH macroemulsions were blended in a ratio of 2.6/1, respectively for 2 minutes in a one liter ESCO mixer equipped with a scraper and rotor/stator high shear device (ESCO-LABOR AG, Switzerland), with only the scraper on at a speed of around 50 to 100 RPM. Produced was a mixture having both high and low pH macroemulsions.

Subsequent to making the high and low pH pre-nanoemulsion mixture and in the same ESCO mixer, the rotor/stator was activated to shear the mixture at a speed of 3000-6000 rpm for up to 5 minutes and until a nanoemulsion with the droplet size (650 nm) was formed (Table 1c) with 1% by weight cystine.

**Table 1c. Nanoemulsion with 1% cystine**

| | wt%* |
|---|---|
| **Aqueous phase** | |
| DI water | Balance |
| NaOH | 0.5 |
| NaCl | 0.3 |
| EDTA | 0.3 |
| Cystine | 1.0 |
| Citric acid | 0.8 |

| **Oil phase** | |
|---|---|
| CCT** | 36.7 |
| DC ES-5300*** | 5.8 |

| | |
|---|---|
| * based on total weight of nanoemulsion (particle size 100 nm) ** Caprylic Capric Triglyceride *** Silicone Emulsifier, Dow Corning | |

### Example 2

Nanoemulsion of Example 2 was combined and mixed with the following ingredients to make the multiple emulsion.

| Multiple Emulsion | |
|---|---|
| Ingredient | Weight % |
| Nanoemulsion * | 55.0 |
| Water | Balance |
| Emulsifier (HLB>8) | 1.7 |
| Thickener | 0.3 |
| EDTA | 0.07 |
| Glycerine | 10 |
| Preservative | 0.1 |

| | |
|---|---|
| * from Example 2 | |

The resulting mixture was sheared with a metal impeller set to 150 rpm under atmospheric pressure and at room temperature (25°C). Recovered was a multiple emulsion having a water particle size of 15 microns. The multiple emulsion was about 45% by weight external aqueous phase, about 24% by weight oil phase and about 31% by weight internal aqueous phase.

### Example 3

The multiple emulsion of Example 2 was stored for three (3) months at 45°C. Surprisingly, no syneresis, coalescence or agglomeration of any kind was observed after Scanning Electron Microscopy of the multiple emulsion. Moreover, viscosity versus shear rate profiles were assessed for multiple emulsion made according to Example 2. It was unexpectedly discovered that while the nanoemulsions of the present invention may comprise actives and/or humectant, the sensory profile of the multiple emulsions of this invention were consistent with an oil-in-water emulsion having less than 3% by weight glycerine or no glycerine.

## Claims

1. A cosmetic composition comprising a multiple emulsion comprising:
(a) an internal phase comprising a water-in-oil nanoemulsion comprising emulsifier, the nanoemulsion having a particle size from 100 to 850 nm, and further comprising from 0.0 to 15% by weight oil soluble benefit agent, from 0.0 to 15% by weight water soluble benefit agent and from 0.0 to 20% by weight humectant based on total weight of the nanoemulsion; and
(b) an external aqueous phase comprising water, from 0.0 to 20% by weight humectant and from 0.0 to 15% by weight water soluble benefit agent based on total weight of the external aqueous phase,
the cosmetic composition comprising from 20 to 65% by weight nanoemulsion based on total weight of the cosmetic composition, with the proviso that the nanoemulsion and external aqueous phase do not simultaneously have 0.0% by weight humectant and in which the water phase of the nanoemulsion of the multiple emulsion, includes water insoluble benefit agents selected from insoluble amino acids selected from phenylalanine, tyrosine, tryptophan, cystine or mixtures thereof; and in which the water insoluble benefit agents make up from 0.02 to 5% by weight of the total weight of the multiple emulsion.

2. The cosmetic composition according to claim 1 wherein the oil phase of the nanoemulsion comprises 0.01 to 15% by weight oil soluble benefit agent.

3. The composition according to claims 1 or 2 wherein the oil soluble benefit agent is Vitamin A, D, E, K, ethylhexylmethoxycinnamate, bis-ethyl hexyloxyphenol methoxyphenol triazine, 2-ethylhexyl-2-cyano-3,3-diphenyl-2-propanoic acid, drometrizole trisiloxane, 3,3,5-trimethyl cyclohexyl 2-hydroxybenzoate, 2-ethylhexyl-2-hydroxybenzoate or a mixture thereof.

4. The composition according to claims 1 to 3 wherein the oil soluble benefit agent is 4-phenylethyl resorcinol, 4-cyclopentyl resorcinol, 4-cyclohexyl resorcinol 4-isopropyl resorcinol, 4-cyclohexyl-5-methylbenzene-1,3-diol, 4-isopropyl-5-methylbenzene-1,3-diol, or a mixture thereof.

5. The composition according to claims 1 to 4 wherein the water phase of the nanoemulsion and/or the external aqueous phase of the multiple emulsion comprises from 0.001 to 10% by weight water soluble benefit agent.

6. The composition according to claim 5 wherein the water soluble benefit agent is arginine, valine, histidine, vitamin B₂, niacinamide (vitamin B₃), vitamin B₆, vitamin C, ascorbyl tetraisopalmitate, magnesium ascorbyl phosphate, ascorbyl glycoside, 4-ethyl resorcinol, sage extract, aloe vera extract, green tea extract, grapeseed extract, thyme extract, chamomile extract, yarrow extract, cucumber extract, liquorice extract, rosemary extract, ensulizole or mixtures thereof.

7. The composition according to claims 1 to 6 wherein the oil soluble benefit agent is an omega-3 fatty acid, omega-6 fatty acid, climbazole, farnesol, ursolic acid, myristic acid, geranyl geraniol, oleyl betaine, cocoyl hydroxyethyl imidazoline, hexanoyl sphingosine, 12-hydroxystearic acid, petroselinic acid, conjugated linoleic acid, terpineol, thymol retinol, retinal, retinyl propionate, retinyl palmitate, retinyl acetate or a mixture thereof.

8. The composition according to claims 1 to 7 wherein water in the nanoemulsion and/or the external aqueous phase comprise from 0.001 to 15% by weight humectant.

9. The composition according to claims 1 to 8wherein the humectant is glycerine.

10. Use of the composition of claim 1 to moisturize skin.

11. Composition according to claims 1 to 9, in which the water-in-oil nanoemulsion of the internal phase of the multiple emulsion is prepared by a process comprising the steps of:
making a first water-in-oil macroemulsion having a pH from 10-14 in order to solubilize the water-insoluble benefit agent; combining the first water-in-oil macroemulsion with a second water-in-oil macroemulsion having a pH from 2.5 to 4 and no benefit agent;
mixing the first and second macroemulsions in a weight ratio of 75:30 to 50:50;
homogenizing or shearing the resulting mixture of macroemulsions to produce an oil continuous nanoemulsion.

## Patentansprüche

1. Kosmetische Zusammensetzung, umfassend eine Mehrfachemulsion, umfassend:
(a) eine innere Phase, umfassend eine Wasser-in-ÖI-Nanoemulsion, umfassend Emulgator, wobei die Nanoemulsion eine Teilchengröße von 100 bis 850 nm aufweist, und ferner umfassend von 0,0 bis 15 Gewichts-% öllösliches Vorteilsmittel, von 0,0 bis 15 Gewichts-% wasserlösliches Vorteilsmittel und von 0,0 bis 20 Gewichts-% Feuchthaltemittel, bezogen auf das Gesamtgewicht der Nanoemulsion, und
(b) eine äußere wässrige Phase, umfassend Wasser, von 0,0 bis 20 Gewichts-% Feuchthaltemittel und von 0,0 bis 15 Gewichts-% wasserlösliches Vorteilsmittel, bezogen auf das Gesamtgewicht der äußeren wässrigen Phase,
wobei die kosmetische Zusammensetzung von 20 bis 65 Gewichts-% Nanoemulsion, bezogen auf das Gesamtgewicht der kosmetischen Zusammensetzung, umfasst, mit der Maßgabe, dass die Nanoemulsion und die äußere wässrige Phase nicht gleichzeitig 0,0 Gewichts-% Feuchthaltemittel aufweisen und in der die Wasserphase der Nanoemulsion der Mehrfachemulsion wasserunlösliche Vorteilsmittel einschließt, ausgewählt aus unlöslichen Aminosäuren, ausgewählt aus Phenylalanin, Tyrosin, Tryptophan, Cystin oder Mischungen davon, und worin die wasserunlöslichen Vorteilsmittel von 0,02 bis 5 Gewichts-% des Gesamtgewichts der Mehrfachemulsion ausmachen.

2. Kosmetische Zusammensetzung nach Anspruch 1, wobei die Ölphase der Nanoemulsion 0,01 bis 15 Gewichts-% öllösliches Vorteilsmittel umfasst.

3. Zusammensetzung nach Anspruch 1 oder 2, wobei das öllösliche Vorteilsmittel Vitamin A, D, E, K, Ethylhexylmethoxycinnamat, Bis-ethyl-hexyloxyphenolmethoxyphenol-triazin, 2-Ethylhexyl-2-cyano-3,3-diphenyl-2-propansäure, Drometrizoltrisiloxan, 3,3,5-Trimethylcyclohexyl-2-hydroxybenzoat, 2-Ethylhexyl-2-hydroxybenzoat oder eine Mischung davon ist.

4. Zusammensetzung nach Anspruch 1 bis 3, wobei das öllösliche Vorteilsmittel 4-Phenylethylresorcin, 4-Cyclopentylresorcin, 4-Cyclohexylresorcin, 4-Isopropylresorcin, 4-Cyclohexyl-5-methylbenzol-1,3-diol, 4-Isopropyl-5-methylbenzol-1,3-diol oder eine Mischung davon ist.

5. Zusammensetzung nach den Ansprüchen 1 bis 4, wobei die Wasserphase der Nanoemulsion und/oder die äußere wässrige Phase der Mehrfachemulsion von 0,001 bis 10 Gewichts-% wasserlösliches Vorteilsmittel umfasst.

6. Zusammensetzung nach Anspruch 5, wobei das wasserlösliche Vorteilsmittel Arginin, Valin, Histidin, Vitamin B₂, Niacinamid (Vitamin B₃), Vitamin B₆, Vitamin C, Ascorbyltetraisopalmitat, Magnesiumascorbylphosphat, Ascorbylglycosid, 4-Ethylresorcin, Salbei-Extrakt, Aloe-Vera-Extrakt, Grüntee-Extrakt, Traubenkern-Extrakt, Thymian-Extrakt, Kamillen-Extrakt, Schafgarben-Extrakt, Gurken-Extrakt, Süßholz-Extrakt, Rosmarin-Extrakt, Ensulizol oder Mischungen davon.

7. Zusammensetzung nach den Ansprüchen 1 bis 6, wobei das öllösliche Vorteilsmittel eine Omega-3-Fettsäure, Omega-6-Fettsäure, Climbazol, Farnesol, Ursolsäure, Myristinsäure, Geranylgeraniol, Oleylbetain, Cocoylhydroxyethylimidazolin, Hexanoylsphingosin, 12-Hydroxystearinsäure, Petroselinsäure, konjugierte Linolsäure, Terpineol, Thymolretinol, Retinal, Retinylpropionat, Retinylpalmitat, Retinylacetat oder eine Mischung davon ist.

8. Zusammensetzung nach den Ansprüchen 1 bis 7, wobei Wasser in der Nanoemulsion und/oder der äußeren wässrigen Phase 0,001 bis 15 Gewichts-% Feuchthaltemittel umfasst.

9. Zusammensetzung nach den Ansprüchen 1 bis 8, wobei das Feuchthaltemittel Glycerin ist.

10. Verwendung der Zusammensetzung nach Anspruch 1 zum Befeuchten der Haut.

11. Zusammensetzung nach den Ansprüchen 1 bis 9, in der die Wasser-in-ÖI-Nanoemulsion der inneren Phase der Mehrfachemulsion durch ein Verfahren hergestellt wird, umfassend die Schritte:
Herstellen einer ersten Wasser-in-ÖI-Makroemulsion mit einem pH-Wert von 10-14, um das wasserunlösliche Vorteilsmittel aufzulösen; Kombinieren der ersten Wasser-in-ÖI-Makroemulsion mit einer zweiten Wasser-in-ÖI-Makroemulsion mit einem pH-Wert von 2,5 bis 4 und keinem Vorteilsmittel; Mischen der ersten und zweiten Makroemulsion in einem Gewichtsverhältnis von 75:30 bis 50:50;
Homogenisieren oder Scheren der resultierenden Mischung der Makroemulsionen, um eine ölkontinuierliche Nanoemulsion herzustellen.

## Revendications

1. Composition cosmétique comprenant une émulsion multiple comprenant :
(a) une phase interne comprenant une nanoémulsion eau-dans-huile comprenant un émulsifiant, la nanoémulsion ayant une taille de particule de 100 à 850 nm, et comprenant de plus de 0,0 à 15 % en masse d'agent bénéfique soluble dans l'huile, de 0,0 à 15 % en masse d'un agent bénéfique soluble dans l'eau et de 0,0 à 20 % en masse d'humectant sur la base de la masse totale de la nanoémulsion ; et
(b) une phase aqueuse externe comprenant de l'eau, de 0,0 à 20 % en masse d'humectant et de 0,0 à 15 % en masse d'agent bénéfique soluble dans l'eau sur la base de la masse totale de la phase aqueuse externe,
la composition cosmétique comprenant de 20 à 65 % en masse de nanoémulsion sur la base de la masse totale de la composition cosmétique, à condition que les nanoémulsion et phase aqueuse externe ne présentent pas simultanément 0,0 % en masse d'humectant et dans laquelle la phase d'eau de la nanoémulsion de l'émulsion multiple, inclut des agents bénéfiques insolubles dans l'eau choisis parmi des acides aminés insolubles choisis parmi la phénylalanine, la tyrosine, le tryptophane, la cystine ou des mélanges de ceux-ci ; et dans laquelle les agents bénéfiques insolubles dans l'eau constituent de 0,02 à 5 % en masse de la masse totale de l'émulsion multiple.

2. Composition cosmétique selon la revendication 1, dans laquelle la phase d'huile de la nanoémulsion comprend de 0,01 à 15 % en masse d'agent bénéfique soluble dans l'huile.

3. Composition cosmétique selon la revendication 1 ou 2, dans laquelle l'agent bénéfique soluble dans l'huile est la vitamine A, D, E, K, l'éthylhexyl méthoxy cinnamate, la bis-éthyl hexyl oxyphénol méthoxyphénol triazine, l'acide 2-éthylhexyl-2-cyano-3,3-diphényl-2-propanoïque, le drométrizole trisiloxane, le 2-hydroxybenzoate de 3,3,5-triméthyl cyclohexyle, le 2-éthylhexyl-2-hydroxybenzoate ou un mélange de ceux-ci.

4. Composition cosmétique selon les revendications 1 à 3, dans laquelle l'agent bénéfique soluble dans l'huile est le 4-phényléthyl résorcinol, 4-cyclopentyl résorcinol, 4-cyclohexyl résorcinol, 4-isopropyl-résorcinol, 4-cyclohexyl-5-méthylbenzène-1,3-diol, 4-isopropyl-5-méthyl-benzène-1,3-diol, ou un mélange de ceux-ci.

5. Composition selon les revendications 1 à 4, dans laquelle la phase d'eau de la nanoémulsion et/ou la phase aqueuse externe de l'émulsion multiple comprend de 0,001 à 10 % en masse d'agent bénéfique soluble dans l'eau.

6. Composition selon la revendication 5, dans laquelle l'agent bénéfique soluble dans l'eau est l'arginine, la valine, l'histidine, la vitamine B₂, le niacinamide (vitamine B₃), la vitamine B₆, la vitamine C, le tétraisopalmitate d'ascorbyle, l'ascorbylphosphate de magnésium, le glycoside d'ascorbyle, le 4-éthyl résorcinol, un extrait de sauge, un extrait d'aloe vera, un extrait de thé vert, un extrait de pépin de raisin, un extrait de thym, un extrait de camomille, un extrait d'achillée, un extrait de concombre, un extrait de réglisse, un extrait de romarin, l'ensulizole ou des mélanges de ceux-ci.

7. Composition selon les revendications 1 à 6, dans laquelle l'agent bénéfique soluble dans l'huile est un acide gras d'oméga-3, acide gras d'oméga-6, le climbazole, le farnésol, l'acide ursolique, l'acide myristique, le géranyl géraniol, l'oléyl bétaïne, la cocoyl hydroxyéthyl imidazoline, l'hexanoyl sphingosine, l'acide 12-hydroxystéarique, l'acide pétrosélinique, l'acide linoléique conjugué, le terpinéol, le thymol rétinol, le rétinal, le propionate de rétinyle, le palmitate de rétinyle, l'acétate de rétinyle ou un mélange de ceux-ci.

8. Composition selon les revendications 1 à 7, dans laquelle l'eau dans la nanoémulsion et/ou la phase aqueuse externe comprend de 0,001 à 15 % en masse d'humectant.

9. Composition selon les revendications 1 à 8, dans laquelle l'humectant est la glycérine.

10. Utilisation de la composition selon la revendication 1 pour hydrater la peau.

11. Composition selon les revendications 1 à 9, dans laquelle la nanoémulsion eau-dans-huile de la phase interne de l'émulsion multiple est préparée par un procédé comprenant les étapes de :
fabrication d'une première macroémulsion eau-dans-huile ayant un pH de 10-14 afin de solubiliser l'agent bénéfique insoluble dans l'eau ; combinaison de la première macroémulsion eau-dans-huile avec une seconde macroémulsion eau-dans-huile ayant un pH de 2,5 à 4 et aucun agent bénéfique ; mélange des première et seconde macroémulsions dans un rapport de masse de 75:30 à 50:50 ; homogénéisation ou cisaillement du mélange résultant de macroémulsions pour produire une nanoémulsion continue dans l'huile.
